(19) Europäisches Patentamt
European Patent Office
Office européen des brevets

(11) EP 2 549 866 B1

(12) EUROPEAN PATENT SPECIFICATION

(45) Date of publication and mention of the grant of the patent:
**24.08.2016 Bulletin 2016/34**

(21) Application number: **11709952.3**

(22) Date of filing: **24.03.2011**

(51) Int Cl.:
***A01N 37/34*** (2006.01) ***A61K 31/277*** (2006.01)
***A61P 33/00*** (2006.01) ***A01N 43/90*** (2006.01)

(86) International application number:
**PCT/EP2011/054534**

(87) International publication number:
**WO 2011/117346 (29.09.2011 Gazette 2011/39)**

# (54) ENDOPARASITICIDAL COMPOSITIONS

ENDOPARASITIZIDE ZUSAMMENSETZUNGEN

COMPOSITIONS ENDOPARASITICIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.03.2010 CH 452102010**

(43) Date of publication of application:
**30.01.2013 Bulletin 2013/05**

(73) Proprietor: **Novartis Tiergesundheit AG**
**4058 Basel (CH)**

(72) Inventors:
- **ROLFE, Peter**
  **Kemps Creek**
  **New South Wales 2171 (AU)**
- **MILLER, Sarah**
  **Kemps Creek**
  **New South Wales 2171 (AU)**
- **REISER, Miriam**
  **CH-4058 Basel (CH)**
- **WIELAND-BERGHAUSEN, Susanne Christine**
  **CH-4058 Basel (CH)**

(74) Representative: **Bassinder, Emma Marie et al**
**Eli Lilly and Company Limited**
**European Patent Operations**
**Erl Wood Manor**
**Sunninghill Road**
**Windlesham, Surrey GU20 6PH (GB)**

(56) References cited:
**WO-A1-2005/044784** **WO-A1-2006/050887**
**WO-A2-02/49641** **WO-A2-2004/000793**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to novel endoparasiticidal compositions and their use in the control of endoparasites, in particular helminths.

There is a continuing need for new agents to manage endoparasitic infestations in livestock animals due to the increasing prevalence of parasites, in particular nematodes, that are resistant to many of the agents currently approved for this indication.

Recently, the chemical class of aminoacetonitrile derivatives (AAD) has been found to be extremely active against endoparasites such as helminths. For example, WO 2005/44784 discloses specific novel members of this chemical class, which offer a new route of combating endoparasitic infestations in livestock animals.

It now has been found, that the combination of a specific member of the class of aminoacetonitrile derivatives with abamectin offers further unexpected advantages. For example, said combination of the two active ingredients broadens the activity spectrum with regard to the endo-parasites in a synergistic manner. Moreover, the number of treatments per year may be reduced significantly. In addiction, the pest resistance management is improved, meaning that the occurrence of resistance against one active ingredient is delayed drastically by the administration of the combination product instead of applying one active ingredient only. Another advantage is that the combination product can be used successfully even in those cases where the worm population has already developed resistance against common anthelmintics.

According, the present invention in one aspect relates to a composition for controlling endoparasites in and on animals, which comprises a combination, in variable proportions, of

(A) a compound of formula (I)

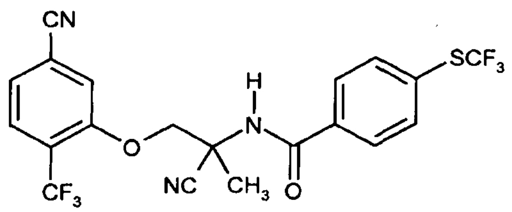

(I),

and

(B) abamectin.

The compound (A) and its synthesis is known, for example, from WO 2005/44784. The compound (A) of the present invention has an asymmetric carbon atom in the 1-position labelled with (1*) in the formula I' below

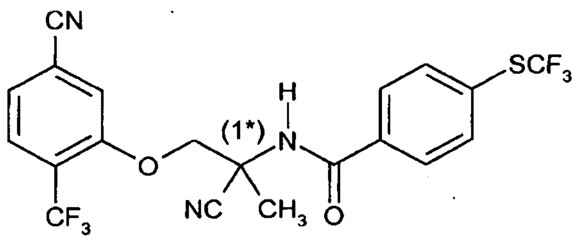

(I').

Accordingly, the compound of formula I may exist as optical isomers. The present invention includes individual enantiomers of the compound of formula I and mixtures thereof, including the racemate. In addition, it has been found that following the separation of the racemate into the two pure enantiomers by standard methods, e. g. by chemical resolution using optically active acid or base or by chromatography on chiral adsorbents, e.g. highpressure liquid chromatography on acetyl cellulose, or by the process as disclosed in WO 2006/50887, one of them has proven to be biologically less active (the distomer), whereas the other enantiomer is highly bioactive (the eutomer). In general, the (1S)-enantiomer of the formula (I') is highly bioactive, whereas the (1 R)-enantiomer is less bioactive.

The compound of formula I may exist in more than one tautomeric form. The present invention encompasses all tautomers, as well as mixtures thereof.

The compound of formula I may be able to form salts with acids or bases. The present invention includes said compound of formula I in form of a salt to the extent that it is pharmaceutically or veterinarily acceptable.

The compound of formula I and their salts may exist in unsolvated or solvated forms. The term solvate herein describes a molecular complex comprising the compound of formula I and one or more pharmaceutically or veterinarily acceptable solvents, for example ethanol or water. In case of water the term "hydrate" is used.

A preferred embodiment of the compound of formula (I) is the enantiomer N-[(1S)-1-cyano-2-(5-cyano-2-trifluoromethylphenoxy)-1-methylethyl]-4-trifluoromethylsulfanylbenzamide.

Component (B) of the compositions of the present invention is abamectin, also named avermectin $B_1$, CAS Registry Number 71751-41-2.

Abamectin is a mixture of two compounds of the formula

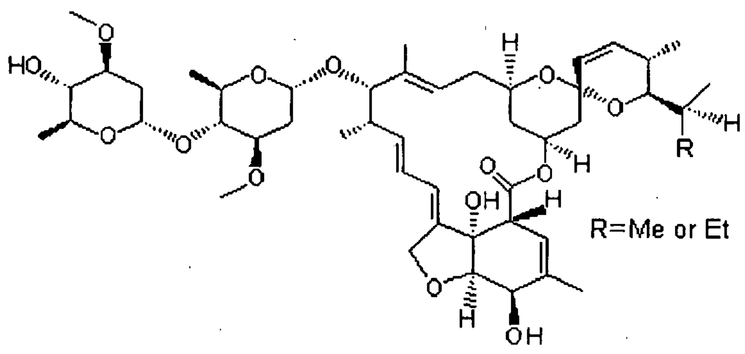

(II),

(i) avermectin $B_{1a}$, the compound of the formula (II) wherein R is ethyl, which is the main compound; and
(ii) avermectin $B_{1b}$, the compound of the formula (II), wherein R is methyl, which is the minor compound.

Abamectin has been registered originally as a mixture of ≥ 80% avermectin $B_{1a}$ and 5 20% $B_{1b}$. Abamectin is known, for example from in US 4,310,519, and is commercially available.

The compositions of the present invention may comprise component (A) and component (B) in any suitable weight ratio, for example in a ratio between 1:100 and 100:1 weight by weight, preferably in a ratio between 1:1 and 50:1 weight by weight, more preferably a ratio between 1:1 and 25:1 weight by weight, and most preferably from 5:1 to 15:1 weight by weight.

Both the aminoacetonitrile compound (A) and the abamectin (B) have an excellent human and animal safety and toxicological profile.

The compositions according to the invention are notable for their broad activity spectrum and are valuable active ingredients for use in pest control, including in particular the control of endoparasites, especially helminths, in and on warm-blooded animals, especially livestock and domestic animals, whilst being well-tolerated by warm-blooded animals and fish.

The compositions of the present invention are effective against helminths, in which the endoparasitic nematodes and trematodes may be the cause of serious diseases of mammals and poultry, e.g. sheep, pigs, goats, cattle, horses, donkeys, dogs, cats, guinea-pigs and exotic birds. Typical nematodes of this indication are: *Haemonchus, Trichostrongylus, Ostertagia, Nematodirus, Cooperia, Ascaris, Bunostonum, Oesophagostonum, Charbertia, Trichuris, Strongylus, Trichonema, Dictyocaulus, Capillaria, Heterakis, Toxocara, Ascaridia, Oxyuris, Ancylostoma, Uncinaria, Toxascaris* and *Parascaris.* The trematodes include, in particular, the family of *Fasciolideae,* especially *Fasciola hepatica.*

It could also be shown surprisingly and unexpectedly that the compositions of the present invention have exceptionally high efficacy against nematodes that are resistant to many active substances. This can be demonstrated *in vitro* by the LDA test and *in vivo* for example in Mongolian gerbils and sheep. It was shown that amounts of active substance which kill sensitive strains of *Haemonchus contortus* or *Trichostrongylus colubriformis,* are also sufficiently effective at controlling corresponding strains that are resistant to benzimidazoles, levamisol and macrocyclic lactones (for example ivermectin) or mixtures thereof.

Certain pests of the species *Nematodirus, Cooperia* and *Oesophagostonum* infest the intestinal tract of the host animal, while others of the species *Haemonchus* and *Ostertagia* are parasitic in the stomach and those of the species *Dictyocaulus* are parasitic in the lung tissue. Parasites of the families *Filariidae* and *Setariidae* may be found in the internal cell tissue and in the organs, e.g. the heart, the blood vessels, the lymph vessels and the subcutaneous tissue. A particularly notable parasite is the heartworm of the dog, *Dirofilaria immitis.* The compounds of formula I are highly effective against these parasites.

The pests which may be controlled by the compositions of the present invention also include those from the class of *Cestoda* (tapeworms), e.g. the families *Mesocestoidae,* especially of the genus *Mesocestoides,* in particular *M. lineatus; Dipylidiidae,* especially *Dipylidium caninum, Joyeuxiella spp.,* in particular *Joyeuxiella pasquali,* and *Diplopy-*

*lidium spp.,* and *Taeniidae,* especially *Taenia pisiformis, Taenia cervi, Taenia ovis, Taeneia hydatigena, Taenia multiceps,Taenia taeniaeformis, Taenia serialis,* and *Echinococcus* spp., most preferably *Taneia hydatigena, Taenia ovis, Taenia multiceps, Taenia serialis; Echinococcus granulosus* and *Echinococcus multilocularis.*

Furthermore, the compositions of the present invention are suitable for the control of human pathogenic parasites. Of these, typical representatives that appear in the digestive tract are those of the genus *Ancylostoma, Necator, Ascaris, Strongyloides, Trichinella, Capillaria, Trichuris* and *Enterobius.* The compounds of the present invention are also effective against parasites of the genus *Wuchereria, Brugia, Onchocerca* and *Loa* from the family of *Dracunculus* and parasites of the genus *Strongyloides* and *Trichinella,* which infect the gastrointestinal tract in particular.

The good endoparasiticidal activity of the compositions of the present invention corresponds to a mortality rate of at least 50-60% of the endoparasites mentioned. In particular, the compositions of the present invention provide a synergistic effect with respect to endoparasiticidal efficacy as shown, for example, in the Examples section. In addition, they are notable for an exceptionally long duration of efficacy.

The compositions of the present invention are employed in unmodified form or preferably together with adjuvants conventionally used in the art of formulation and may therefore be processed in a known manner to give, for example, emulsifiable concentrates, directly dilutable solutions, dilute emulsions, soluble powders, powder mixtures, granules or microencapsulations in polymeric substances. As with the compositions, the methods of application are selected in accordance with the intended objectives and the prevailing circumstances.

The formulation, i.e. the agents, preparations or compositions containing the active ingredients (A) and (B) and optionally a solid or liquid adjuvant, are produced in a manner known *per se,* for example by intimately mixing and/or grinding the active ingredients with the adjuvants, for example with solvents, solid carriers and/or surface-active compounds (surfactants).

The solvents in question may be: alcohols, such as ethanol, propanol or butanol; glycols and their ethers and esters, such as propylene glycol, dipropylene glycol ether, ethylene glycol, ethylene glycol monomethyl or -ethyl ether, propylene glycol monocaprylate, propylene glycol dicaprylate, propylene glycol dicaprate; ketones, such as cyclohexanone, isophorone or diacetanol alcohol; strong polar solvents, such as N-methyl-2-pyrrolidone, dimethyl sulfoxide or dimethylformamide; water; vegetable oils, such as rape oil, castor oil, coconut oil, or soybean oil; silicone oils; or mixtures of two or more of the above-mentioned solvents.

Suitable surfactants are, for example, non-ionic surfactants, such as, for example, nonylphenolpolyethoxyethanols; castor oil polyglycol ethers, for example macrogol glycerolhydroxystearate 40; polyethylene glycols; polypropylene/polyethylene oxide adducts; or fatty acid esters of polyoxyethylene sorbitan, e.g. polyoxyethylene sorbitan monooleate.

Preferred application forms for usage on warm-blooded animals in the control of helminths comprise solutions; emulsions including classical emulsions, microemulsions and self-emulsifying compositions, that are waterless organic, preferably oily, compositions which form emulsions - together with body fluids - upon addition to an animal body; suspensions (drenches); food additives; powders; tablets including effervescent tablets; boli; capsules including microcapsules; and pour-on formulations; whereby the physiological compatibility of the formulation excipients must be taken into consideration. Particularly preferred application forms are emulsions or solutions, in particular emulsions and especially self-emulsifying organic compositions containing no water or low amounts of water only.

The binders for tablets and boli may be chemically modified polymeric natural substances that are soluble in water or in alcohol, such as starch, cellulose or protein derivatives (e.g. methyl cellulose, carboxymethyl cellulose, ethylhydroxyethyl cellulose, proteins such as zein, gelatin and the like), as well as synthetic polymers, such as polyvinyl alcohol, polyvinyl pyrrolidone etc. The tablets also contain fillers (e.g. starch, microcrystalline cellulose, sugar, lactose etc.), glidants and disintegrants.

If the anthelminthics are present in the form of feed concentrates, then the carriers used are e.g. performance feeds, feed grain or protein concentrates. Such feed concentrates or compositions may contain, apart from the active ingredients, also additives, vitamins, antibiotics, chemotherapeutics or other pesticides, primarily bacteriostats, fungistats, coccidiostats, or even hormone preparations, substances having anabolic action or substances which promote growth, which affect the quality of meat of animals for slaughter or which are beneficial to the organism in another way. If the compositions or the active ingredients (A) and (B) contained therein are added directly to feed or to the drinking troughs, then the formulated feed or drink contains the active ingredients preferably in a concentration of ca. 0.0005 to 0.02 % by weight (5-200 ppm).

As a rule, the anthelminthic compositions according to the invention contain 0.1 to 99 % by weight, especially 0.1 to 95 % by weight of active ingredients (A) and (B), 99.9 to 1 % by weight, especially 99.8 to 5 % by weight of a solid or liquid admixture, including 0 to 25 % by weight, especially 0.1 to 25 % by weight of a surfactant.

A preferred composition according to the invention comprises 0.5 to 5 % w/v of a compound of formula (I), 0.01 to 0.5 % w/v abamectin, 5 to 30 % w/v surfactants and ad 100% w/v of organic solvents. A more preferred composition according to the invention comprises 1.0 to 5 % w/v of a compound of formula (I), 0.1 to 0.5 % w/v abamectin, 10 to 20 % w/v surfactants and ad 100% w/v of organic solvents.

Application of the compositions according to the invention to the animals to be treated may take place, for example, topically, perorally, parenterally or subcutaneously, the composition being present, for example, in the form of a solution, emulsion, suspension, (drench), powder, tablet, boli, capsule or pour-on- or spot-on formulation. A preferred embodiment of the invention relates to compositions for parenteral use or, in particular, for peroral use.

Whereas it is preferred to formulate commercial products as concentrates, the end user will normally use dilute formulations.

Such compositions may also contain further additives, such as stabilisers, antioxidants, for example tocopherols like α-tocopherol, anti-foaming agents, viscosity regulators, binding agents, colors or tackifiers, as well as other active ingredients, in order to achieve special effects. Preferably, the composition comprises from 0.001 to 1 % w/v of one or more antioxidants. If desired, the formulations of the present invention may comprise a color, for example in an amount of from 0.001 to 1 % w/v.

Anthelminthic compositions of this type, which are used by the end user, similarly form a constituent of the present invention.

In each of the processes according to the invention for pest control or in each of the pest control compositions according to the invention, the active ingredients of formula I can be used in all of their steric configurations or in mixtures thereof.

The compositions of the present invention may also be used for prophylactically protecting warm-blooded animals, especially productive livestock, domestic animals and pets, against parasitic helminths, in particular by administering the active ingredients of the invention or the active ingredient formulations prepared therefrom to the animals as an additive to the feed, or to the drinks or also in solid or liquid form, orally or by injection or parenterally.

The following Examples illustrate the invention further.

Preparation of an oral formulations comprising N-((1S)-1-cyano-2-(5-cyano-2-trifluoro-methylphenoxy)-1-methylethyl)-4-trifluoromethylsulfanylbenzamide (monepantel) or abamectin or a mixture of monepantel and abamectin

Solutions for oral application are prepared by intimately mixing the active ingredient(s) with solvents (propylene glycol monocaprylate, propyleneglycol dicaprylate/dicaprate, propylene glycol), surfactants (macrogolglycerol hydroxystearate, polysorbate 80), antioxidant (α-tocopherol) and color (β-carotene). The final composition is as follows.

| | |
|---|---|
| Monepantel | 2.5 % w/v |
| Abamectin | 0.2 % w/v |
| Propylene glycol | 2.0 % w/v |
| macrogolglycerol hydroxystearate | 5.0 % w/v |
| polysorbate 80 | 10.00 w/v |
| propylene glycol monocaprylate | 20.0 % w/v |
| β-carotene | 0.06 % w/v |
| α-tocopherol | 0.05 % w/v |
| propyleneglycol dicaprylate/dicaprate | ad 100 % w/v |

In-vivo test on Cooperia curticei on sheep using peroral application

The study concerned a blinded parallel-group efficacy study against the L4 stage of the subject nematodes. The experimental induced worm burdens of monepantel/abamectin combination treated animals were compared to those of an untreated control group and sheep treated animals with either monepantel or abamectin mono-formulations.

To this end the required larvae were generated first of all through culturing of characterized field isolates. Each animal then received the same dose of L3 (infective) larvae, and their larvae burdens were allowed time to mature. Once the larvae reached the L4 stage, the sheep were allocated to treatment groups 1, 2, 3 and 4 and treated as defined in the Table below:

Oral application using the above described formulations:

| Group. | Treatment | Dose mg/kg bodyweight | Number of animals |
|---|---|---|---|
| 1 | Untreated control | - | 8 |
| 2 | Monepantel | 0.625 | 8 |
| 3 | Abamectin | 0.05 | 8 |

(continued)

| Group. | Treatment | Dose mg/kg bodyweight | Number of animals |
|---|---|---|---|
| 4 | Monepantel & Abamectin | 0.625 & 0.05 | 8 |

Two weeks after treatment (days 14-17) the sheep were slaughtered, their gastro-intestinal tracts recovered and processed for enumeration of total worm burdens. The primary measure of efficacy was calculated from the mean worm reduction in groups 2-4 when compared to the untreated control group 1. Efficacies were calculated using the formula

$$\text{Efficacy [\%]} = 100 \times (C - T) / C$$

wherein C and T are the worm count means fo the Control and Treated groups, respectively.

The following values were obtained:

| Group | Treatment | Worm count [Geometric mean value] | Efficacy [%] |
|---|---|---|---|
| 1 | Untreated control | 4266 | - |
| 2 | Monepantel | 1453 | 66.0 |
| 3 | Abamectin | 1352 | 68.3 |
| 4 | Monepantel & Abamectin | 170 | 96.0 |

A two-way ANOVA model having the following effects
- dose of Monepantel
- dose of Abamectin, and
- the interaction of the two doses;

was used to assess a synergistic effect of the combination product. To this end, worm counts were log transformed as follows:

| Group | Treatment | log [Worm count] [geometric mean value] | Reduction log [worm count] | Reduction worm count [factor] |
|---|---|---|---|---|
| 1 | Untreated control | 3.631 | - | - |
| 2 | Monepantel | 3.165 | -0.466 | ~ 2.9 |
| 3 | Abamectin | 3.134 | -0.497 | ~ 3.1 |
| 4 | Monepantel & Abamectin | 2.256 | -1.375 | ~ 23 |

With no interaction between the two active ingredients, worm count reduction of Group 4 would have been expected to amount to a factor of 2.9x3.1 = ~ 9. The value actually observed, factor 23, is significantly higher (p = 0.0070) and thus indicative of a synergistic effect.

**Claims**

**1.** A composition for controlling endoparasites in and on animals, which comprises a combination of

(A) a compound of formula (I)

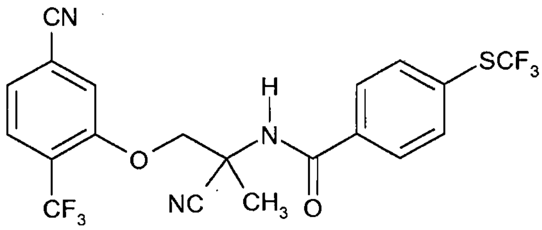

(I),

and

(B) abamectin.

**2.** A composition according to claim 1, wherein component (A) is the (1S)-enantiomer of the compound of formula I'

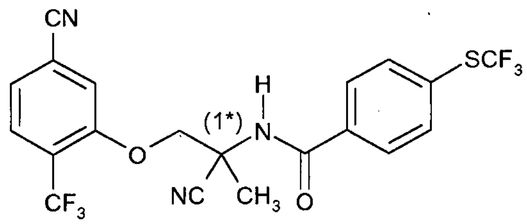

(I').

**3.** A composition according to claim 1 or 2, comprising the components (A) and (B) in a ratio between 1:1 and 50:1 weight by weight.

**4.** A composition according to claim 3, comprising the components (A) and (B) in a ratio between 5:1 and 15:1 weight by weight.

**5.** A composition according to claim 1 or 2, comprising 0.5 to 5% w/v of a compound of formula (I), 0.01 to 0.5% w/v abamectin, 5 to 30% w/v of one or more surfactants and ad 100% w/v of organic solvents.

**6.** A composition according to claim 1 or 2, comprising 1.0 to 5 % w/v of a compound of formula (I), 0.1 to 0.5 % w/v abamectin, 10 to 20 % w/v of one or more surfactants and ad 100% w/v of organic solvents.

**7.** A composition according to any one of claims 1 to 6, which is for peroral or parenteral use.

**8.** A composition according to any one of claims 1 to 6 for use in controlling endoparasites in and on warm-blooded animals.

**Patentansprüche**

**1.** Zusammensetzung zur Bekämpfung von Endoparasiten in und auf Tieren, wobei sie eine Kombination aus

(A) einer Verbindung der Formel (I)

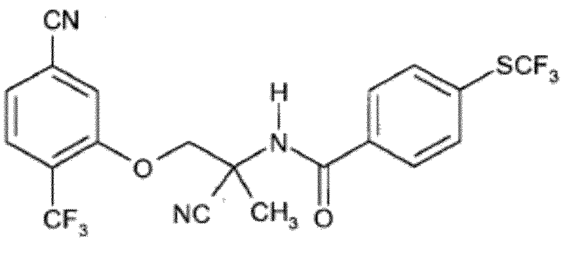

(I), und

(B) Abamectin

umfasst.

**2.** Zusammensetzung nach Anspruch 1, wobei es sich bei der Verbindung (A) um das (1S)-Enantiomer der Verbindung der Formel I' handelt

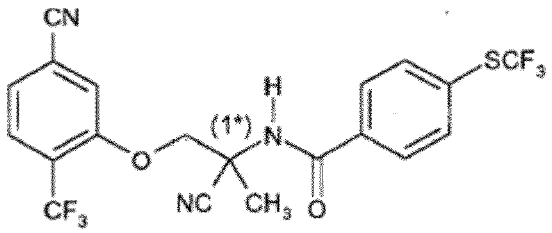

(I').

**3.** Zusammensetzung nach Anspruch 1 oder 2, welche die Bestandteile (A) und (B) in einem Verhältnis zwischen 1:1 und 50:1 jeweils nach Gewicht umfasst.

**4.** Zusammensetzung nach Anspruch 3, welche die Bestandteile (A) und (B) in einem Verhältnis zwischen 5:1 und 15:1 jeweils nach Gewicht umfasst.

**5.** Zusammensetzung nach Anspruch 1 oder 2, welche 0,5 bis 5 % (w/v) einer Verbindung der Formel (I), 0,01 bis 0,5 % (w/v) an Abamectin, 5 bis 30 % (w/v) eines oder mehrerer Tenside und den zu 100 % (w/v) fehlenden Anteil an organischen Lösungsmitteln umfasst.

**6.** Zusammensetzung nach Anspruch 1 oder 2, welche 1,0 bis 5 % (w/v) einer Verbindung der Formel (I), 0,1 bis 0,5 % (w/v) an Abamectin, 10 bis 20 % (w/v) eines oder mehrerer Tenside und den zu 100 % (w/v) fehlenden Anteil an organischen Lösungsmitteln umfasst.

**7.** Zusammensetzung nach einem beliebigen der Ansprüche 1 bis 6, die zur peroralen oder parenteralen Verwendung bestimmt ist.

**8.** Zusammensetzung nach einem beliebigen der Ansprüche 1 bis 6, zur Verwendung bei der Bekämpfung von Endoparasiten in und auf warmblütigen Tieren.

## Revendications

**1.** Composition pour lutter contre des endoparasites dans et sur des animaux, qui comprend une combinaison de

(A) un composé de formule (I)

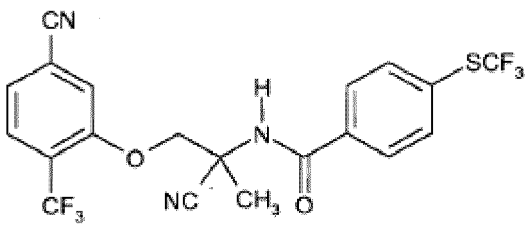

(I),

et
(B) abamectine.

**2.** Composition selon la revendication 1, dans laquelle le constituant (A) est l'énantiomère (1 S) du composé de formule I'

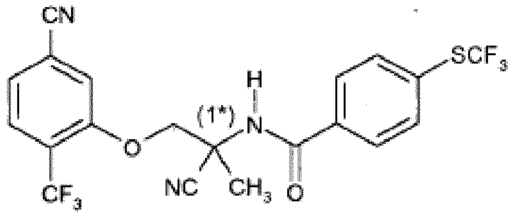

(I').

**3.** Composition selon la revendication 1 ou 2, comprenant les constituants (A) et (B) dans un rapport compris entre 1:1 et 50:1 en poids par poids.

**4.** Composition selon la revendication 3, comprenant les constituants (A) et (B) dans un rapport compris entre 5:1 et 15:1 en poids par poids.

**5.** Composition selon la revendication 1 ou 2, comprenant 0,5 à 5 % en p/v d'un composé de formule (I), 0,01 à 0,5 % en p/v d'abamectine, 5 à 30 % en p/v d'un ou de plusieurs tensioactifs et ad 100 % en p/v de solvants organiques.

**6.** Composition selon la revendication 1 ou 2, comprenant 1,0 à 5 % en p/v d'un composé de formule (I), 0,1 à 0,5 % en p/v d'abamectine, 10 à 20 % en p/v d'un ou de plusieurs tensioactifs et ad 100 % en p/v de solvants organiques.

**7.** Composition selon l'une quelconque des revendications 1 à 6, qui est destinée à une utilisation perorale ou parentérale.

**8.** Composition selon l'une quelconque des revendications 1 à 6 pour une utilisation dans la lutte contre des endoparasites dans et sur des animaux à sang chaud.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200544784 A
- WO 200650887 A
- US 4310519 A